Europäisches Patentamt

European Patent Office

Office européen des brevets

(19)

(11) **EP 0 725 770 B1**

(12) **EUROPEAN PATENT SPECIFICATION**

(45) Date of publication and mention
of the grant of the patent:
**05.01.2000 Bulletin 2000/01**

(51) Int Cl.[7]: **C07C 2/58**

(86) International application number:
**PCT/EP94/03588**

(21) Application number: **94931024.7**

(22) Date of filing: **27.10.1994**

(87) International publication number:
**WO 95/11872 (04.05.1995 Gazette 1995/19)**

(54) **PROCESS FOR UPGRADING A PARAFFINIC FEEDSTOCK**

VERFAHREN ZUR QUALITÄTSVERBESSERUNG EINER PARAFFINISCHEN BESCHICKUNG

PROCEDE D'ENRICHISSEMENT D'UNE CHARGE D'ALIMENTATION PARAFFINIQUE

(84) Designated Contracting States:
**AT BE CH DE DK ES FR GB GR IT LI NL SE**

(30) Priority: **29.10.1993 EP 93203044**

(43) Date of publication of application:
**14.08.1996 Bulletin 1996/33**

(73) Proprietor: **SHELL INTERNATIONALE
RESEARCH MAATSCHAPPIJ B.V.
2596 HR Den Haag (NL)**

(72) Inventors:
 • **VAN BRUGGE, Paulus, Theodorus, Maria
NL-1031 CM Amsterdam (NL)**
 • **DE GROOT, Christoffel
NL-1031 CM Amsterdam (NL)**
 • **MESTERS, Carolus, Matthias, Anna, Maria
NL-1031 CM Amsterdam (NL)**
 • **DE VRIES, Auke, Fimme
NL-1031 CM Amsterdam (NL)**

(56) References cited:
**EP-A- 0 565 197          US-A- 2 315 129
US-A- 2 355 339          US-A- 3 851 004**

**Description**

The present invention relates to a process for upgrading a paraffinic feedstock. More specifically the invention relates to a process for alkylation of a paraffinic feedstock by the condensation of paraffins with olefins.

The production of highly branched hydrocarbons such as trimethylpentane is important by virtue of their use as gasoline blending components of high octane number. Traditional production of highly branched hydrocarbons is by condensation of isobutane with light olefins, usually butenes but sometimes mixtures of propene, butenes and possibly pentenes using large quantities of conventional strong liquid acid catalysts, such as hydrofluoric or sulphuric acids. An emulsion of immiscible acid and hydrocarbon is agitated to emulsify the catalyst and reactant and refrigerated to control the highly exothermic reaction. By fine control of a complex interrelation of process variables high quality alkylate production may be maintained. The acid is recycled after use. It is desirable to use a process which is less hazardous and toxic and environmentally more acceptable.

Processes have been proposed to overcome these problems by using solid acids as catalysts. However paraffin olefin condensation yields both desired alkylate and undesired oligomerisation product. When catalysing alkylation with solid acids it has been found that hydrocarbon deposit formation causes progressive deactivation of the catalyst. Regeneration techniques are however known for removal of hydrocarbonaceous deposits from solid acid catalysts to restore catalyst activity, typically by increasing the temperature of the catalyst and oxidising the deposits. Nevertheless rapid deactivation of the solid acid catalysts compared with liquid acid catalysts is a disadvantage.

It is known from US-A-2,355,339 that gaseous hydrogen at a pressure of from 50 pounds, preferably of about 50 to 500 or 1000 pounds has an impact on the life of a catalyst to produce high anti-knock, chiefly saturated, branch chain hydrocarbons within the gasoline boiling point range from paraffins and olefins.

It is further known from US-A-3,851,004 that liquid solutions of hydrogen in a saturated hydrocarbon can be used to restore alkylation activity. It is specifically stated in said document that the catalyst restoration procedure should be carried out after residual olefin feed has been removed from the catalyst bed in order to prevent loss of olefin due to its being hydrogenated during the subsequent hydrogen treatment of the catalyst composition.

There is a need for an environmentally acceptable alkylation process which selectively yields highly branched alkylate at an acceptable rate for prolonged periods on stream.

Copending European patent application numbers 92201015 and 92201016 disclose processes for upgrading a paraffinic feedstock comprising supplying feedstock and olefin at specified paraffin to olefin ratio to a reactor containing a solid acid catalyst, preferably a zeolite beta catalyst and subsequently removing the upgraded product wherein the processes are operated at high olefin conversion in respectively internal and external circulation reactors having respectively a high variance in residence time distribution of liquid reactor contents and a high extent of external circulation of liquid reactor contents. It has been found that under these conditions alkylation products are obtained for a catalyst lifetime greater than that using known solid acid catalysed processes.

It has now surprisingly been found that condensation of paraffins with olefins may be achieved using a solid acid catalysed alkylation process achieving an acceptable rate of production of product alkylate at high selectivity wherein the process is operated under conditions adapted to discourage the deactivation of the catalyst by hydrocarbonaceous deposits. It has moreover been found that the condensation may be carried out for longer periods on stream to breakthrough of unreacted olefins in the reactor effluent than is possible using other solid acids. Alternatively operation may be carried out by using the present catalyst at a greater rate of production of product alkylate than for other solid acids for the same regeneration duty. it is surprisingly found that the conditions employed do not deleteriously affect the quality of the product alkylate as might be expected.

Accordingly the present invention provides a process for upgrading a paraffinic feedstock comprising contacting feedstock and olefin at a paraffin to olefin ratio greater than 5 v/v with a solid acid catalyst characterised in that the process is carried out at a temperature less than 150°C in the presence of a hydrogen source dissolved in a reactive paraffin, and a hydrogenating metal component.

Reference herein to hydrocarbonaceous deposits is to compounds or radicals formed during the catalysed condensation of paraffin with olefin(s), which compounds or radicals are thought to have a semi-permanent deactivating effect on the catalyst. It is speculated that these deposits comprise oligomerisation products and fragments thereof formed and retained within the zeolite pores. In the absence of an understanding of the nature of the deposits and their manner of association it is not possible to systematically approach the problem of inhibiting their formation or their deactivating effect, or of effecting their removal in a manner non-disruptive to the condensation reaction.

Reference herein to alkylate is to a mixed condensation product of isoparaffin with olefin(s), and by the term oligomerisation product is meant a condensation product of a plurality of olefin molecules. Alkylate is characterised by a higher motor octane number (MON) than oligomerisation product. Typically alkylate comprising highly branched paraffins with 5 to 12 carbon atoms has a MON of 86 or above, for example in the range 90 to 94 whilst a corresponding oligomerisation product may comprise a mixture of paraffinic and olefinic hydrocarbons typically of MON of less than 85, for example in the range 80 to 82.

A hydrogen source in combination with hydrogenating metal component is used so as to achieve transfer of hydrogen to the catalytic environment such that a hydrocarbonaceous effluent is obtained derived from oligomerisable fragments which would otherwise associate to form a hydrocarbonaceous deposit. Without wishing to be limited to a particular theory it would appear that conditions which do not effectively achieve transfer of hydrogen to the catalytic environment would result in substantially no effluent being obtained derived from the oligomerisable fragments. It would also appear that effective transfer takes place under conditions such that fragments are selectively hydrogenated at a rate in excess of oligomerisation thereof, thereby significantly reducing the rate of adsorption as hydrocarbonaceous deposit on the catalyst, and reducing the rate of deactivation of the catalyst. The rate of hydrogenation would therefore be appropriately controlled by the nature of the hydrogen source and hydrogenating metal component and concentration of hydrogen available to take part in hydrogenation, for a given rate of formation of oligomerisable fragments during the condensation reaction. The efficiency of the hydrogenation may be determined from the composition of the effluent which preferably comprises lower saturated hydrocarbons derived from the oligomerisable fragments.

It is surprisingly found that in the presence of a hydrogen source in combination with hydrogenating metal component substantially no reduction in the yield of alkylate takes place. It is believed that the process of the present invention operates by selective hydrogenation of species other than desired reactants.

A hydrogenating metal component may be selected from a Group VIII or Group IB metal component, combinations thereof and combinations with other components for example Group VIB metal components, the hydrogenating metal component being chemically associated with the solid acid catalyst, for example by ion exchange, admixed with the catalyst as separate particles on a suitable carrier or being brought into physical association with the catalyst for the duration of the condensation reaction. Preferably a hydrogenating metal component comprises metal components selected from platinum, palladium, copper and nickel, combinations thereof and combinations with additional metal components selected from tungsten and molybdenum, in metal, ion or alloy form. Preferably the hydrogenating metal component is incorporated in the catalyst. Suitably the hydrogenating metal component is present in an amount of 0.005-10.0 wt%, preferably 0.01-1.0 wt%, more preferably 0.05-0.5 wt% of solid acid catalyst present.

The hydrogen source may be, for example, hydrogen gas dissolved in a paraffinic reactant employed in the condensation, for example with a liquid lower alkane comprised in the paraffinic feedstock. The hydrogen source may be present in a ratio of 99:1 to 1:99 v/v in the paraffinic reactant.

In a further embodiment the present invention relates to a cyclic process for catalytically upgrading a paraffinic feedstock and upgrading the activity of deactivated catalyst comprising the steps a) of supplying feedstock and olefin at a paraffin to olefin ratio greater than 5 v/v to a reactor containing a solid acid catalyst and removing effluent comprising upgraded product and b) of exposing the catalyst to a medium for upgrading the activity of the catalyst with removal of hydrocarbonaceous effluent wherein the step a) is carried out in accordance with the present invention.

Preferably the medium for upgrading the activity of the catalyst is any known medium for regeneration of solid acid catalysts. More preferably the medium is a hydrogenating medium as described in copending European patent application No. 93202515.8, the contents of which application are incorporated herein by reference.

The process of the present invention may be carried out in any suitable reactor or reactor configuration. The process may be operated in batchwise or in continuous manner whereby regeneration of the catalyst in known manner for the regeneration of solid acid conversion catalysts is carried out continuously or on breakthrough of olefin reactant in the reactor effluent or build up in the reactor. Operation in a fixed bed or slurry phase reactor is preferred, optionally employing external or internal circulation of liquid reactor contents in step a). Multiple reactors may be operated in series with respect to feed and effluent lines for improved process efficiency. Preferably the process is operated in continuous manner advantageously enabling continuous production of alkylate. Suitably, a plurality of reactors or reactor beds can be operated with feed and effluent lines in parallel whereby step a) and step b) are operated in antiphase in at least two beds. For example multiple fixed or slurry phase reactors may be operated with parallel feed and effluent lines such as in a swing bed system such that alkylate is continuously produced from one or another catalyst bed, and catalyst is regenerated as appropriate in at least one parallel bed.

Alternatively a slurry phase reactor may be operated continuously in the condensation reaction, for example by use of a continuous regeneration system whereby a proportion of catalyst is continuously or periodically withdrawn from the slurry reactor to a separate vessel for operation of a suitable regeneration step (step b)) and returned to the slurry reactor for operation of the condensation reaction (step a)).

Effluent comprising the hydrogenated oligomerisable fragments may be retained in the system and recycled with effluent comprising unreacted feedstock and alkylate from the condensation reaction. Preferably the combined effluent is distilled prior to recycle, whereby the concentration of alkylate in the recycle stream may be controlled. Alternatively a portion of the effluent is separated by non-selective means and recycled. The effluent comprising hydrogenated oligomerisable fragments may find use in chemical applications, in which case selective separation from the main effluent is preferred.

Addition of the hydrogen source containing paraffin may be continuous or batchwise with continuous or batchwise withdrawal of effluent, the frequency of batchwise introduction or the continuous flow rate selected to maintain the available hydrogen which may contact the catalyst within a required range for the selective hydrogenation of oligomerisable fragments. A sample of continuously or batchwise withdrawn effluent or of the reaction vessel fluid contents is conveniently monitored for indication of selectivity of hydrogenation. For example alkylate content of hydrocarbonaceous effluent components may be monitored to determine level of condensation of olefins or hydrogenated oligomerisable fragments content may be monitored to ensure hydrogenation conditions are prevailing. Suitable techniques for monitoring effluent composition are known in the art such as gas chromatographic separation techniques in combination with flame ionisation detection techniques.

A suitable flow rate or residence time of hydrogen source-containing paraffin may be determined to attain the selectivity of hydrogenation according to the present invention, taking into consideration i.a concentration or partial pressure of available hydrogen and nature and concentration of hydrogenating metal component with respect to the wt% of (zeolite) crystals in the catalyst, choice of operation in batchwise or continuous mode and age of catalyst, nature of catalyst composition, and the conversion conditions such as olefin space velocity, extent if any of paraffin circulation and the paraffin to olefin ratio.

By the process of the invention it is possible to operate at an acceptable selectivity, i.e. obtaining an acceptable alkylate yield for the duration of the time on stream. It would appear that the catalyst alkylation activity is upheld throughout partial deactivation and until full deactivation of all sites has occurred, at which point olefin breakthrough is observed. Hence for the duration of time on stream it would appear that hydrocarbonaceous deposits remain associated with the acid sites of the catalyst and are not detected in the product. In order to maintain a higher production rate it may be advantageous to operate to a predetermined production rate corresponding to partial deactivation of catalyst in combination with a high frequency of deposit removal by regeneration.

A preferred operation of the process of the invention is in the preparation of highly branched paraffins containing 5 to 12 carbon atoms, preferably containing 5,6,7,8,9 or 12 carbon atoms, most preferably trimethyl-butane, -pentanes or -hexanes. The process may be applied in the upgrading of a paraffinic feedstock comprising iso-paraffins having from 4 to 8 carbon atoms as desired, suitably of a feedstock comprising isobutane, 2-methylbutane, 2,3-dimethylbutane, 3-methylhexane or 2,4-dimethylhexane, most preferably a paraffinic feedstock comprising iso-paraffins having 4 or 5 carbon atoms. Suitable feedstocks for the process of the invention include iso-paraffin containing fractions of oil conversion products such as naphtha fractions, and refined iso-paraffin feedstocks such as refined iso-butane.

The olefins containing stream suitably comprises a lower olefin containing hydrocarbon which may optionally contain additional non-olefinic hydrocarbons. Suitably the olefins containing stream comprises ethene, propene, iso- or 1- or 2-(cis or trans) butene or pentene optionally diluted for example with propane, iso-butane, n-butane or pentanes. The process of the invention may suitably be operated downstream of a fluid catalytic cracking unit, an MTBE etherification unit or an olefin isomerisation unit.

Preferably the process of the invention is conducted in such a manner as to control initial contact of the catalyst with olefin feed. Suitably the reactor is first charged with paraffinic feedstock. Internal or external circulation may advantageously be operated as described in copending European patent application numbers 92201015 and 92201016 for the high dilution of olefin which is continuously and quantitatively charged at a paraffin to olefin ratio greater than 5 v/v to the reactor at an acceptable olefin space velocity and with desired hydrogen source-containing paraffin co-feed. Suitable olefin space velocity is such that conversion is maintained in excess of 90%. The feed mixture may advantageously be charged in known manner for improved dispersion via a plurality of inlet ports.

For transition from step a) to step b) in a reactor or bed operated in batchwise process, the supply of olefin is terminated or is switched to a feed line operated in parallel supplying a bed operated in antiphase with respect to the cyclic process. Remaining alkylate entrained in the reactor or bed is removed, for example by terminating supply of paraffinic feedstock and drying the reactor or bed at elevated temperature and/or reduced pressure, or by continuing supply of hydrocarbonaceous feedstock as purge for remaining alkylate in the reactor whereafter the supply of hydrocarbonaceous feedstock is also terminated. The catalyst is then contacted with hydrogenating medium, for example by feeding hydrogenating medium continuously with optional recycle or for a period to attain a desired partial pressure in the reactor as above described.

For transition from step a) to step b) of a process operated with use of a slurry reactor and continuous catalyst regeneration, catalyst is periodically or continuously withdrawn from the reactor vessel and passed to a separate vessel for operation of step b) in which hydrogenating medium is periodically or continuously introduced and effluent withdrawn accordingly. Removal of entrained effluent from step a) may be effected within the hydrogenation vessel by drying at elevated temperature and/or reduced pressure or by subjecting catalyst prior to hydrogenation with a non-olefinic hydrocarbonaceous purge for removal of entrained alkylation effluent. In a continuous withdrawal process, the purge may be carried out in a separate vessel prior to passing catalyst to the hydrogenation vessel.

For repeat of the batchwise cyclic process, the supply of paraffinic feedstock is recommenced for a period to saturation of the catalyst, whereafter the supply of mixed paraffinic feedstock and olefin is recommenced as above described and hydrogenating conditions recommenced. For reuse in step a) of catalyst withdrawn from a slurry reactor for operation of step b), saturation with paraffinic feedstock may be carried out in a separate vessel prior to returning catalyst to the reactor.

Where it is desired to operate with internal or external circulation for example as described in copending application numbers 92201015 and 92201016, the extent thereof may be selected according to the desired frequency and extent of removal of hydrocarbonaceous deposit.

It may be desirable to convert alkylate further to other products. Accordingly the process of the invention may be operated with use of a feed line for introducing alkylate to a further process step.

The process of the invention is carried out at a temperature less than 150 °C, for example at 60 to 100 °C, preferably at 70 to 80 °C, for example at 75 °C. Reactor pressure may be between 1 and 40 bar, preferably between 10 and 30 bar. Suitably reactor pressure is just greater than the vapour pressure of the paraffin content of the feedstock at the reactor temperature so as to maintain the reaction in the liquid phase.

Paraffin to olefin ratio is suitably in excess of 5 v/v, preferably in the range of 10 to 50 v/v, more preferably in the range of 12 to 30 v/v, most preferably in the range of 15 to 30 v/v. A low paraffin to olefin ratio suitably within the range 5 to 10 v/v favours the formation of higher alkylates, such as isoparaffins containing 12 carbon atoms. Operation at high paraffin to olefin ratios favours the formation of lower alkylates but may incur increased effluent distillation and external circulation costs.

Preferred olefin conversion is at least 95%, more preferably at least 98%, more preferably at least 99%, such as for example 99.5, 99.8 or 99.9%. Most preferably olefin conversion is substantially complete, i.e substantially 100%.

Preferred operating ranges of olefin space velocity include from 0.05 to 10.0 kg/kg.hr, preferably from 0.1 to 5.0 kg/kg.hr most preferably from 0.2 to 1.0 kg/kg.hr.

Suitably hydrogen source-containing paraffin is added throughout the condensation reaction to provide available hydrogen in an amount equal to 0.01 to 1.0 mol/mol, preferably 0.03 to 0.1 mol/mol, more preferably 0.04 to 0.06 mol/mol for example 0.05 mol/mol with respect to olefin present. The amount of available hydrogen provided is dependent on the nature of the prevailing hydrogenation conditions, such as the nature of a hydrogenating metal component associated with the catalyst. The amount of hydrogen source-containing paraffin supplied should in any event be sufficient to hydrogenate oligomerisable fragments formed in the reaction mixture at a rate superior to the rate of oligomerisation thereof, but without formation of a secondary phase consisting mainly of hydrogen in the reaction vessel. Preferably hydrogen is co-fed as a solution in the isobutane feedstock supply to the reactor.

The process may advantageously be operated by monitoring of composition of effluent using known means for example as hereinbefore described whereby feed rates and feed lines are adapted in response to multivariable constraint control of a selected operating parameter. In a preferred operation of the process of the invention the effluent is monitored during operation and composition readings fed to an advanced process controller operating on a process model to achieve optimum process efficiency.

The process of the invention may be operated by use of any solid acid catalyst. Particularly suitable catalysts include strongly acidic wide pore molecular sieves such as zeolites of pore diameter greater than 0.70nm, sulphate mounted metal oxides, amorphous silica alumina, metal halide on alumina, macroreticular acid cation exchange resins such as acidified perfluorinated polymers, heteropoly acids and activated clay minerals.

Examples of zeolite catalysts include zeolite beta, zeolite omega, mordenite and faujasites, particularly zeolites X and Y, of which zeolite beta is most preferred. Zeolites may be ion exchanged for example with an ammonium source and optionally calcined to promote acidity. Examples of sulphate mounted metal oxides include those of zirconium oxide, titanium oxide and iron oxide. Preferred is sulphate mounted zirconium oxide. Examples of amorphous silica alumina include acidified such as fluorinated amorphous silica alumina. Examples of metal halides on alumina include hydrochloric acid treated aluminium chloride and aluminium chloride on alumina. Examples of cation exchange resins include Nafion (Trade Mark) an acidified perfluorinated polymer of sulphonic acid. Examples of heteropoly acids include $H_3PW_{12}O_{40}$, $H_4SiMo_{12}O_{40}$ and $H_4SiW_{12}O_{40}$. Examples of clay minerals include smectite and montmorillonite.

Preferably a catalyst comprises zeolite beta crystals conforming to the structural classification of a zeolite beta, for example as in Newman, Treacy et al., Proc. R. Soc. London Ser. A 420, 375 or in US 3,308,069, first reported synthesis. Zeolite beta may be prepared using tetramethylammonium hydroxide as a template and as described in Zeolites 8 (1988) 46-53. The zeolite is suitably calcined before use, for example at 550 °C for 2 hours. The desired silicon to aluminium ratio of the calcined material may be selected as appropriate. The zeolite may be ion exchanged for example with ammonium nitrate and calcined to give the hydrogen form to promote acidity.

In a preferred embodiment of the invention the zeolite is further ion exchanged with a hydrogenating metal component as above defined, preferably selected from platinum, palladium, copper and nickel, combinations thereof and combinations with additional metal components selected from tungsten and molybdenum, in an amount of 0.005 - 10 %wt on zeolite, preferably 0.01 - 1 %wt, more preferably 0.05-0.5 %wt, and subsequently calcined. Preferably the zeolite is subject to reducing conditions prior to use to render the hydrogenating metal component in metal form. Ion exchange with metal components as above defined has been found to provide catalysts giving beneficial selective hydrogenation when employed in appropriate combination with hydrogen source-containing paraffin during the condensation reaction.

The zeolite may be employed directly or further combined with a support. For example the zeolite may be present in combination with a refractory oxide that serves as binder material such as silica, alumina, silica-alumina, magnesia, titania, zirconia and mixtures thereof. Alumina is specially preferred. The weight ratio of refractory oxide and zeolite suitably ranges from 10:90 to 90:10, preferably from 50:50 to 15:85. The binder is suitably combined with zeolite prior to final calcination thereof, the combination optionally extruded and finally calcined for use.

The process is now illustrated by way of examples.

EXAMPLE 1

Catalyst A comprising zeolite beta was prepared using tetramethylammonium hydroxide according to a recipe as described in Zeolites 8 (1988) 46-53. After synthesis the solids were separated from the liquid, washed with deionised water, dried and calcined at 550C for 2 hours. The silicon to aluminium ratio of the calcined material was 14.7. The zeolite was brought in the acidic form by ion exchange with ammonium nitrate and calcining for 2 hours at 450 °C.

Catalysts B, C and D were prepared by loading samples of Catalyst A with 0.1 %wt palladium, copper and nickel respectively by ion exchange with aqueous solutions of palladium tetraamine chloride, copper nitrate and nickel nitrate respectively and finally calcining for 2 hours at 450 °C.

EXAMPLE 2

60 - 140 mesh size catalyst B was dried for 2 hours at 200 °C in air then loaded into a fixed bed recycle reactor. The reactor was pressure tested with hydrogen at room temperature, raised to a temperature of 250 °C and maintained at 250 °C for 16 hours in a flow of hydrogen. The reactor was brought to a temperature for alkylation reaction maintained at about 90 °C. Isobutane was fed to the reactor to saturation. An effluent stream was withdrawn with continued feed of isobutane and a mixture of 2-butene and isobutane feedstock (paraffin olefin ratio of 30 v/v) was co-fed with hydrogen in a ratio to hydrogen to olefins of around 0.05 mol/mol, into the reactor at an olefin space velocity of 0.2 kg/kg.h with recycle of reactor effluent at a recycle ratio of around 250. The effluent withdrawn from the reactor was analysed on-line by GLC and separated into an atmospheric liquid and a vapour product. Reaction was continued until butene breakthrough observed in the effluent.

The reaction was repeated substantially as above described with Catalysts C and D in place of Catalyst B.

Olefin conversion was greater than 99.0 mol%, i.e substantially complete. The results are given in Table 1 below. Yield of essentially paraffinic C5+ product was 200% by weight on olefin.

COMPARATIVE EXAMPLE

The reaction was also repeated for purpose of comparison substantially as above described but with the following modifications whereby the reactions were not carried out in accordance with the present invention, firstly with use of catalyst A in place of catalyst B and secondly with use of catalyst B but without co-feed of hydrogen. The results are given in Table 1 below.

TABLE 1

| Catalyst | Hydrogenating condition | Lifetime to olefin breakthrough (hours) |
|---|---|---|
| B | Pd/$H_2$ | 52 |
| C | Cu/$H_2$ | 32 |
| D | Ni/$H_2$ | 31 |
| A | $H_2$ | 25 |
| B | Pd | 25 |

TABLE 2

| Analysis of the alkylate produced with catalyst B (Table 1) | |
|---|---|
| Alkylate | Yield (%wt) fraction |
| $C_5$/$C_5$+ | 6 |
| $C_6$/$C_5$+ | 5 |

TABLE 2   (continued)

| Analysis of the alkylate produced with catalyst B (Table 1) | |
|---|---|
| Alkylate | Yield (%wt) fraction |
| $C_7/C_5+$ | 6 |
| $C_8/C_5+$ | 79 |
| $C_9+/C_5+$ | 4 + |
| | $\overline{100}$ |

From Example 2 it is clear that the lifetime of the catalysts B to D is significantly extended with respect to that of Catalyst A (no hydrogenation metal component). Moreover, lifetime of the catalysts B to D is extended by addition of hydrogen to the condensation reaction. The yield of C5+ indicates that no deterioration is detected in the alkylate yield due to the hydrogenating conditions employed.

**Claims**

1.  Process for upgrading a paraffinic feedstock comprising contacting feedstock and olefin at a paraffin to olefin ratio greater than 5 v/v with a solid acid catalyst characterised in that the process is carried out at a temperature less than 150 degrees C in the presence of a hydrogen source dissolved in a reactive paraffin and a hydrogenating metal component.

2.  Process according to claim 1, wherein the hydrogen source comprises hydrogen gas.

3.  Prccess according to claim 1 or claim 2, wherein the hydrogenating metal component is selected from a Group 1B or VIII metal component, combinations thereof and combinations with an additional component selected from a Group VI-B metal component.

4.  Process according to claim 3, wherein the hydrogenating metal component comprises one or more metal components selected from platinum, palladium, copper and nickel, combinations thereof and combinations with additional metal components selected from tungsten and molybdenum, in metal, ion or alloy form.

5.  Process according to any one of claims 1 to 4, wherein the hydrogenating metal component is chemically or physically associated or admixed with the catalyst.

6.  Process according to any one of claims 1 to 5, wherein the hydrogenating metal component is present in an amount of 0.005-10.0 wt% of solid acid catalyst present.

7.  Process according to claim 6, wherein the hydrogenating metal component is present in an amount of 0.01-1.0 wt% of solid acid catalyst present.

8.  Process according to claim 7, wherein the hydrogenating metal component is present in an amount of 0.05-0.5 wt% of solid acid catalyst present.

9.  Process according to any one of claims 1 to 8, wherein the catalyst comprises a component selected from a zeolite of pore diameter greater than 0.70 nm, sulphate mounted metal oxide, amorphous silica-alumina, metal halide on alumina, macroreticular acid cation exchange resin, heteropoly acid and activated clay mineral.

10. Process according to any one of claims 1 to 9, wherein the catalyst comprises a zeolite beta component.

11. Process according to any one of claims 1 to 10, carried out in a fixed bed or slurry phase reactor optionally employing external or internal circulation of liquid reactor contents.

12. Process according to any one of claims 1 to 11, comprised in a cyclic process for catalytically upgrading a paraffinic feedstock and upgrading the activity of deactivated catalyst comprising the steps a) of carrying out the process according to claim 1 and removing effluent comprising upgraded product and b) of exposing the catalyst to a medium for upgrading the activity of the catalyst with removal of hydrocarbonaceous effluent.

13. Process according to claim 12, wherein the reactor comprises a fixed bed or slurry phase reactor optionally employing external or internal circulation of liquid reactor contents in step a).

14. Process according to claim 12 or claim 13, carried out in a plurality of reactors or reactor beds operated with feed and effluent lines in parallel whereby step a) and step b) are operated in antiphase in at least two beds.

15. Process according to any one of claims 12 to 14, carried out in a slurry reactor wherein catalyst is withdrawn continuously or periodically from the reactor to a separate vessel for operation of step b) and returned to the reactor for operation of step a).

**Patentansprüche**

1. Verfahren zur Aufbesserung eines paraffinischen Einsatzmaterials, umfassend das Inkontaktbringen von Einsatzmaterial und Olefin bei einem Volumenverhältnis von Paraffin zu Olefin von größer als 5 mit einem festen sauren Katalysator, dadurch gekennzeichnet, daß das Verfahren bei einer Temperatur unter 150°C in Gegenwart einer in einem reaktionsfähigen Paraffin gelösten Wasserstoffquelle und einer hydrierenden Metallkomponente ausgeführt wird.

2. Verfahren nach Anspruch 1, worin die Wasserstoffquelle Wasserstoffgas umfaßt.

3. Verfahren nach Anspruch 1 oder 2, worin die hydrierende Metallkomponente aus einer Gruppe IB- oder Gruppe VIII-Metallkomponente, Kombinationen hievon und Kombinationen mit einer weiteren, unter einer Gruppe VIB-Metallkomponente ausgewählten Komponente ausgewählt ist.

4. Verfahren nach Anspruch 3, worin die hydrierende Metallkomponente eine oder mehrere Metallkomponenten, ausgewählt unter Platin, Palladium, Kupfer und Nickel, deren Kombinationen und Kombinationen mit weiteren, unter Wolfram und Molybdän ausgewählten Metallkomponenten, in metallischer, ionischer oder Legierungsform umfaßt.

5. Verfahren nach einem der Ansprüche 1 bis 4, worin die hydrierende Metallkomponente chemisch oder physikalisch mit dem Katalysator assoziiert oder vermischt ist.

6. Verfahren nach einem der Ansprüche 1 bis 5, worin die hydrierende Metallkomponente in einer Menge von 0,005 bis 10,0 Gew.-%, bezogen auf den vorliegenden festen sauren Katalysator, zugegen ist.

7. Verfahren nach Anspruch 6, worin die hydrierende Metallkomponente in einer Menge von 0,01 bis 1,0 Gew.-%, bezogen auf den vorliegenden festen sauren Katalysator, zugegen ist.

8. Verfahren nach Anspruch 7, worin die hydrierende Metallkomponente in einer Menge von 0,05 bis 0,5 Gew.-%, bezogen auf den vorliegenden festen sauren Katalysator, zugegen ist.

9. Verfahren nach einem der Ansprüche 1 bis 8, worin der Katalysator eine unter einem Zeolith mit einem Porendurchmesser von größer als 0,70 nm, sulfathältigem Metalloxid, amorphem Siliziumoxid-Aluminiumoxid, Metallhalogenid auf Aluminiumoxid, makroretikularem saurem Kationenaustauscherharz, Heteropolysäure und aktiviertem Tonmineral ausgewählte Komponente umfaßt.

10. Verfahren nach einem der Ansprüche 1 bis 9, worin der Katalysator eine beta-Zeolithkomponente umfaßt.

11. Verfahren nach einem der Ansprüche 1 bis 10, das in einem Festbettreaktor oder Slurryphasenreaktor ausgeführt wird, gegebenenfalls unter Anwendung eines externen oder internen Kreislaufs von flüssigem Reaktorinhalt.

12. Verfahren nach einem der Ansprüche 1 bis 11, enthalten in einem zyklischen Verfahren zum katalytischen Aufbessern eines paraffinischen Einsatzmaterials und Verbessern der Aktivität von deskativiertem Katalysator, umfassend die Stufen a) des Durchführens des Verfahrens nach Anspruch 1 und des Abnehmens eines Abstroms, der aufgebessertes Produkt enthält, und b) des Einwirkenlassens eines Mediums zur Steigerung der Aktivität des Katalysators auf den Katalysator, unter Abtrennung eines kohlenwasserstoffhältigen Abstroms.

**13.** Verfahren nach Anspruch 12, worin der Reaktor einen Festbettreaktor oder einen Slurryphasenreaktor umfaßt, gegebenenfalls unter Anwendung von externem oder internem Kreislauf von flüssigem Reaktorinhalt in Stufe a).

**14.** Verfahren nach den Ansprüchen 12 oder 13, das in einer Mehrzahl von Reaktoren oder Reaktorbetten ausgeführt wird, die mit parallelen Speise- und Abstromleitungen betrieben werden, wobei die Stufen a) und b) in wenigstens zwei Betten in Antiphase betrieben werden.

**15.** Verfahren nach einem der Ansprüche 12 bis 14, das in einem Slurryreaktor ausgeführt wird, wobei der Katalysator kontinuierlich oder periodisch aus dem Reaktor in ein getrenntes Gefäß abgezogen wird, um die Stufe b) auszuführen, und zum Reaktor zur Ausführung von Stufe a) zurückgeführt wird.

## Revendications

**1.** Procédé d'amélioration d'une charge d'alimentation paraffinique comprenant la mise en contact de la charge d'alimentation et d'une oléfine à un rapport paraffine à oléfine supérieur à 5 volumes/volume avec un catalyseur d'acide solide, caractérisé en ce que le procédé est réalisé à une température inférieure à 150°C en présence d'une source d'hydrogène dissoute dans une paraffine réactive et d'un composant métallique d'hydrogénation.

**2.** Procédé suivant la revendication 1, dans lequel la source d'hydrogène comprend du gaz d'hydrogène.

**3.** Procédé suivant l'une ou l'autre des revendications 1 et 2, dans lequel le composant métallique d'hydrogénation est choisi parmi un composant de métal du Groupe IB ou VIII, leurs combinaisons et les combinaisons avec un composant additionnel choisi parmi un composant de métal du Groupe VIB.

**4.** Procédé suivant la revendication 3, dans lequel le composant métallique d'hydrogénation comprend un ou plusieurs composants métalliques choisis parmi le platine, le palladium, le cuivre et le nickel, leurs combinaisons et les combinaisons avec des composants métalliques additionnels choisis parmi le tungstène et le molybdène, sous la forme de métal, d'ion ou d'alliage.

**5.** Procédé suivant l'une quelconque des revendications 1 à 4, dans lequel le composant métallique d'hydrogénation est chimiquement ou physiquement associé ou mélangé au catalyseur.

**6.** Procédé suivant l'une quelconque des revendications 1 à 5, dans lequel le composant métallique d'hydrogénation est présent en une quantité de 0,005-10,0% en poids du catalyseur d'acide solide présent.

**7.** Procédé suivant la revendication 6, dans lequel le composant métallique d'hydrogénation est présent en une quantité de 0,01-1,0 % en poids du catalyseur d'acide solide présent.

**8.** Procédé suivant la revendication 7, dans lequel le composant métallique d'hydrogénation est présent en une quantité de 0,05-0,5 % en poids du catalyseur d'acide solide présent.

**9.** Procédé suivant l'une quelconque des revendications 1 à 8, dans lequel le catalyseur comprend un composant choisi parmi une zéolite d'un diamètre de pore supérieur à 0,70 nm, un oxyde métallique fixé sur sulfate, une silice-alumine amorphe, un halogénure métallique sur alumine, une résine d'échange de cations d'acide macroréticulaire, un hétéropolyacide et une argile minérale activée.

**10.** Procédé suivant l'une quelconque des revendications 1 à 9, dans lequel le catalyseur comprend un composant de zéolite bêta.

**11.** Procédé suivant l'une quelconque des revendications 1 à 10, réalisé dans un réacteur à lit fixe où à phase en suspension éventuellement en utilisant une circulation externe ou inteme de contenu de réacteur liquide.

**12.** Procédé suivant l'une quelconque des revendications 1 à 11, compris dans un procédé cyclique pour améliorer catalytiquement une charge d'alimentation paraffinique et améliorer l'activité d'un catalyseur désactivé comprenant les étapes a) de réalisation du procédé suivant la revendication 1 et de séparation d'effluent comprenant le produit amélioré et b) d'exposition du catalyseur à un milieu pour améliorer l'activité du catalyseur avec séparation de l'effluent hydrocarboné.

13. Procédé suivant la revendication 12, dans lequel le réacteur comprend un réacteur à lit fixe ou à phase en suspension éventuellement en utilisant une circulation exteme ou inteme de contenu de réacteur liquide dans l'étape a).

14. Procédé suivant l'une ou l'autre des revendications 12 et 13, réalisé dans une série de réacteurs ou de lits de réacteur fonctionnant avec des conduits d'alimentation et d'effluent en parallèle de sorte que l'étape a) et l'étape b) sont réalisées en antiphase dans au moins deux lits.

15. Procédé suivant l'une quelconque des revendications 12 à 14, réalisé dans un réacteur à suspension dans lequel le catalyseur est extrait de façon continue ou périodique du réacteur vers un récipient séparé pour la réalisation de l'étape b) et renvoyé au réacteur pour la réalisation de l'étape a).